Europäisches Patentamt

European Patent Office

Office européen des brevets

(11) Publication number: **0 169 232**
**B1**

(12) **EUROPEAN PATENT SPECIFICATION**

(45) Date of publication of patent specification: **25.04.90**

(21) Application number: **85900781.7**

(22) Date of filing: **18.01.85**

(86) International application number:
**PCT/NO85/00003**

(87) International publication number:
**WO 85/03231 01.08.85 Gazette 85/17**

(51) Int. Cl.⁵: **A 61 K 49/02**

(54) Composition for technetium-99m labelling of proteinaceous material.

(30) Priority: **23.01.84 SE 8400325**

(43) Date of publication of application:
**29.01.86 Bulletin 86/05**

(45) Publication of the grant of the patent:
**25.04.90 Bulletin 90/17**

(84) Designated Contracting States:
**AT BE CH DE FR GB LI NL SE**

(56) References cited:
**EP-A-0 063 002**
**EP-A-0 078 642**
**EP-A-00 968 71**
**SE-B-79 020 095**
**US-A-4 027 005**
**US-A-4 094 965**
**US-A-4 313 928**
**US-A-4 416 865**

(73) Proprietor: **INSTITUTT FOR ENERGITEKNIKK**
**P.O. Box 40**
**N-2007 Kjeller (NO)**
(73) Proprietor: **SUNDREHAGEN, Erling**
**Langbakken 3**
**N-1430 As (NO)**

(72) Inventor: **INSTITUTT FOR ENERGITEKNIKK**
**P.O. Box 40**
**N-2007 Kjeller (NO)**
Inventor: **SUNDREHAGEN, Erling**
**Langbakken 3**
**N-1430 As (NO)**

(74) Representative: **Lambert, Hugh Richmond et al**
**D. YOUNG & CO. 10 Staple Inn**
**London, WC1V 7RD (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The present invention is concerned with compositions for technetium-99m labelling of proteins or cells for the detection and scintigraphic depiction of pathological or non-pathological processes in living organisms including man and in individuals in which such proteins or cells participate in the processes or accumulate in the regions of the organisms where the processes take place. The invention can also be used in diagnosis of inflammation, infection, graft tissue rejection and thrombosis, particularly such diagnostic methods using $^{99m}$Tc.

Compositions for the labelling of proteins and cells with radionuclides have become important for medical and biological diagnostics and research. Labelling with nuclides having radiation and decay characteristics enabling external detection and depiction of the processes and diseases of the organisms, are particularly advantageous. Radioactivity labelled immunoglobulins having affinity for antigens in tissue, cells, molecules in biological fluids and microorganisms are used for detection and depiction of cancer diseases and infectious disease processes. Radioactive indium and iodine labelled leukocytes, such as granulocytes and lymphocytes, are used for the detection and depiction of inflammation and rejection reactions. Radioactively labelled blood platelets are used for the detection and depiction of thromboembolic diseases.

Various types of radionuclides have hitherto been used for such purposes. Iodine 131 and 125 have been used for the labelling of both proteins and cells, but the suitability for detection and depiction of processes in the human body is restricted because of the less advantageous radiation and decay characteristics of these nuclides. Iodine 123 has more advantageous radiation and decay characteristics, but the use of this nuclide is restricted by its high cost and limited accessibility because the preparation of this nuclide requires a cyclotron or other particle accelerator. Chromium 51 has been used for such purposes but has radiation characteristics which are not suitable for external detection and depiction. Indium 111 has a disintegration rate and radiation acceptable for such uses, but requires relatively high radiation doses to enable the examination of a patient or organism. Also a cyclotron of other particle accelerator is necessary for the preparation of indium 111, which restricts the accessibility of the nuclide and causes a high price.

The use of technetium-99m for the above described purposes will result in considerably lower radiation doses to patients or other organisms which are examined because its nuclide half life is 6.02 hours and only 13% of the radiation is non-penetrating diagnostically not exploitable radiation. In other words 87% of the radiation from $^{99m}$Tc is gamma radiation which can be scintigraphically detected. Only 13% of the radiation is non-gamma or gamma at a different energy level so as to make it non-useful for radiographic diagnosis. The half life is sufficiently long to permit use of $^{99m}$Tc, but short enough so that the patient is not needlessly subjected to large doses of radiation after the radiographic testing. However, the shorter half life will make it necessary to prepare the technetium-99m compositions immediately before their use, i.e. shortly before the introduction into the blood system.

The nearly one and only form of technetium-99m which is available commercially is the pertechnetate. A sterile pertechnetate-99m solution is obtained by means of a $^{99m}$Tc/$^{99}$Mo generator, which gives a considerably better yield for technetium-99m than for the other previously noted nuclides.

However, for most of the technetium-99m labelled compounds, the 7-valent technetium-99m present in the pertechnetate must be reduced to lower valencies. This reduction can very simply and effectively be carried out by means of divalent tin ions. Thus, it has been possible to prepare aqueous solutions of $^{99m}$Tc complexes with albumin, plasmin, immunoglobulins and other proteins by means of stannous chloride as a reductant. However, a portion of the 99m technetium-99m in the solutions complex with the stannous ions to form technetium-99m/tin radiocolloids.

Since both the desired $^{99m}$Tc/protein complex and the undesired $^{99m}$Tc/tin complex formation reactions are reversible, the portion of $^{99m}$Tc complexed with the tin becomes particularly high at low concentrations of protein. Carboxylic acids or carboxylates, for example tartrate and citrate, have been added to the solution to lower the portion of $^{99m}$Tc tied up in the $^{99m}$Tc/tin radiocolloid. The carboxylates are added to complex with the tin ions to remove them from the solution and thereby minimize the formation of the $^{99m}$Tc/tin complex. The stannous ions in the tin/carboxylate complex are still effective as reducing agents for the technetium (VII) ions. However, the carboxylate ion also complexes with the technetium-99m ions and competes with the proteins so that radiochemically pure technetium-99m/protein complex is difficult to obtain. In fact, while labelling of proteins by this method has been reported in the literature, the quality of such labelling has not been adequate to permit use in medical applications.

The portion of technetium-99m/tin radiocolloid can also be reduced by reducing the concentration of tin in the solution. However, there is a limit as to how low this concentration can be without loss of the necessary reduction potential for the reduction of the pertechnetate, particularly if the reducing agent is stored in the form of a solution, in frozen form, or as a solid substance from lyophilization of the solution.

Technetium-99m has been used to complex with phosphorus containing anions for use in skeletal scintigraphy. As discussed by Andrew J. Tofe et al in their abstract published at the Second International Symposium on Radiopharmaceuticals in March 1979, the stannous ion concentration becomes unacceptably low when used in skeletal scintigraphy due to the presence of oxidants in the standard solution. These oxidants are either radiolytically induced or introduced through chemical contamination. Ascorbic acid has therefore been used as stabilizer for the divalent tin ions. As stated by Tofe et al 2,5-dihydroxybenzoic, acid may be used as a stabilizer for the divalent tin ions in preparation kits for technetium-99m complexes with phosphorus-containing anions for skeleton scintigraphy. In fact, they state that ascorbic acid and 2,5-dihydroxy-benzoic acids are equivalents. The formation of complexes is not disclosed.

European Patent Specification EP—A—0,078,462 discloses the use of stabilizing agents for reduced 99m-Tc and stannous ions, the stabilizing agent being an organic compound having an amine group and a carboxylic acid group attached to an aromatic ring. A preferred stabilizing agent is the sodium salt of 4-aminobenzoic acid (PABA). The stabilizer reduces or prevents decomposition of radiopharmaceutical compositions on standing and is chosen to avoid the risk that the stabilizing agent may complex the reduced technetium.

European Patent Specification EP—A—0,096,871 discloses the use of complexes of radioactive indium, technetium, gallium or ruthenium with a substituted pyridine having an electron-donating or withdrawing group on a carbon adjacent to the ring nitrogen and having, independently, hydrogen, methoxy, ethoxy, methyl, halogen, ethyl, carboxy or carboxymethyl on the other four carbons. The production of these complexes involves complicated operations including extraction with chloroform and evaporation.

Technetium-99m complexes with phosphate-containing anions have been proposed for technetium-99m labelling of cells, such as erythrocytes, and leukocytes. Technetium-99m phosphorus colloids have been used for labelling of phagocytic cells, but the phagocytosis of colloidal particles has, however, turned out to alter the biological and physical characteristics of the cells.

There have been reports of the successful labelling of immunoglobulins which have an affinity for cancer cells with radioactive indium and radioactive iodine. However, there have been no reports of the successful labelling of such immunoglobulins with $^{99m}$Tc, or of the successful labelling of immunoglobulins with specific affinity for leukocytes such as granulocytes or lymphocytes.

U.S. Patent Specification US—A—4,027,005 relates to technetium complexes with particular polyhydroxycarboxylic acids and their derivatives. Acids of the benzoic acid group are not mentioned, and the intended use of the agents are not as labelling agent for proteins or cells, for example, but as chemically stable diagnostic complexes on their own for the depiction of kidneys and infarct (for example heart infarct). There is no teaching or suggestion of dihydroxy-benzoic acid and/or any use as a labelling agent for proteins or cells.

Swedish Acceptance Print 431,286 (New England Nucles Corporation) pertains to labelling of aggregated protein, i.e. not protein in solution or proteins in cells. A series of stabilizers is mentioned, such as phosphonate, phosphate and aminocarboxylate. The compositions and use differ from the present invention, so that the previously known technique would not suggest the present invention.

The present invention provides a chemical composition for use in $^{99m}$Tc, labelling of protein or cells comprising proteins or cells to be labelled, the amount of protein as such or in the form of cell protein being 0.25 to 10 mg/ml, from 0.5 to 50 mmol/l of a benzoic acid derivative that is an aminobenzoic, monohydroxybenzoic or dihydroxybenzoic acid or a water-soluble salt thereof, and a stannous salt in an amount to provide 4 to 150 μmol/l of stannous ions. The intended use of the composition is *inter alia* for detection and depiction of processes in human beings and other biological organisms, both for research and diagnostics.

The composition may be in the from of a ready-to-use mixture with which proteins or cells are subjected to radioactive labelling with technetium-99m by means of complex-formation, the amount of technetium-99m being 0.5 to 30 mCi/ml. Complexes are not formed under the conditions specified by Tofe et al (supra).

The benzoic acid derivative is advantageously 2,3-, 2,5- or 3,4-dihydroxybenzoic acid, or 4-amino-benzoic, acid, or 2-, 3- or 4-hydroxybenzoic acid.

Preferably 2,5-dihydroxybenzoic acid or a salt thereof is used as dihydroxybenzoic acid. This is because 2,5-dihydroxybenzoic acid is metabolite of aspirin.

The reduction of pertechnetate ions is effected by means of divalent tin, in an aqueous solution of the other ingredients.

The reduction is advantageously carried out by means of a stannous salt solution having a pH and ionic strength depending on the proteins or cells which are to be labelled. The pH and ionic strength are chosen to achieve optimum viability of the cell types or minimum denaturation of the proteins which are to be labelled which is known in the art. It is important to note that selection of pH and ionic strength is made in each individual case in view of the characteristics of the specific protein or cell types which are to be labelled.

Preferably a stannous halide or a stannous salt of an organic carboxylic acid is used as the stannous salt.

In the present invention two effects of the benzoic acid derivatives are exploited, i.e. as weak complexing agents to keep Tc and stannous ion in solution (avoidance of radiocolloids) to mediate formation of Tc-protein or Tc-cell labelling, and as antioxidant stabilizer to preserve the stannous ions.

In the preparation of the composition, the aqueous solution of stannous salt, benzoic acid derivative and, proteinaceous material, is freeze-dried for future reconstitution to aqueous solution in combination with the aqueous pertechnetate solution.

In more detail, the process of preparing a sterile consists of preparing a sterile aqueous solution of pertechnetate and an aqueous solution of the other components of the composition, i.e., the benzoic acid derivative or a water soluble salt thereof and a stannous salt. The resulting benzoic acid derivative tin solution is adjusted to suitable pH value and ionic strength, corresponding to a pH value and ionic strength tolerated by the added proteins or cells which are to labelled. For low pH ranges, about 2 to about 5, stannous chloride is advantageously used as the stannous salt.

For more neutral pH ranges, greater than about 5, the stannous ions will be hydrolyzed and precipitated as hydroxides, which can be avoided by using stannous carboxylates as stannous citrate or stannous tartrate as the stannous salt. The amount of stannous salt can further be kept at a very low level because of the antioxidizing characteristic of the benzoic acid derivative. At pH greater than 5, sodium or other tartrate or citrate salt may be added to bring the stannous ion to tartrate or citrate ion ratio to 1:1 to 1:25, preferably 1:1 to 1:5 and more preferably 1:1 to 1:25.

The prepared solution is then sterilized, preferably by filtration. The sterile aqueous solution may be given prolonged shelf life by complete removal of water, prefearbly by freeze-drying to a solid substance which can be stored for months.

Ready-to-use compositions are achieved by simply mixing together the solutions or mixing the pertechnetate solution with the mixture containing the remaining components in dried solid form, which may be carried out immediately before the composition being put to its intended use. For optimum labelling yield, the mixture of the solution of labelling reagents and the proteins or cells to be labelled should be left to stand for some time, such as 5 to 45 minutes and preferably 15 to 20 minutes, before the preparations are put to their intended use.

For the practical purposes, it is desirable to include the protein as a portion of the aqueous solution containing the benzoic acid derivative and tin stannous salt described above, advantageously as constituents of the solution being freeze-dried or dried in another way.

For the achievement of increased radiochemical purity, the technetium-99m labelled cells which are obtained should be removed from the aqueous solution in which they are suspended during the labelling and be washed or separated in a solution having ionic strength an pH tolerated by the cell, for example blood plasma or buffer solutions compatible with the cells in question.

The technique of the present invention also enables the radioactive labelling of immunoglobulins, particularly those with specific affinity for leukocytes, including granulocytes and lymphocytes. By labelling such immunoglobulins, leukocytes may be indirectly labelled with technetium-99m by mixing the labelled immunoglobulins with blood from a patient in vitro or by injecting the immunoglobulin solution into the patient to label the leukocytes in vivo.

Since the concentration of the technetium-99 solution injected into each patient is extremely low (the total amount of technetium is in the order of $10^{12}$ atoms), it is not possible to actually determine the nature of the technetium-99/protein complex which is formed. Further, it is not possible to extrapolate the technetium reactions by observing higher concentrations of $^{99}TC$, While not intending to be bound, it is believed that the benzoic acid derivative forms a "weak" complex with the reduced technetium-99m. This "weak" benzoic acid derivative/technetium-99m complex is sufficiently strong to prevent formation of the technetium-99m/tin complex and thereby deters formation of the technetium-99/tin complex. However, the technetium-99m/protein complex is sufficiently stronger than the dihydroxy-benzoic acid/technetium-99m complex so that the dihydroxy-benzoic acid/technetium-99m complex does not deter formation of the protein complex. In fact, the benzoic acid derivative complex facilitates the protein complex formation by effectively keeping the technetium-99m available for formation of the protein complex.

While Tofe et al. have suggested the use of dihydroxybenzoic acid in the formation of phosphorus containing complexes, stating that dihydroxy-benzoic acid is equivalent to ascorbic acid, Tofe et al. used the dihydroxy-benzoic acid solely as a stabilizer to deter oxidation of the stannous ions. Further, with respect to the technetium-99m/protein complexes, ascorbic acid is not equivalent to dihydroxybenzoic acid. In fact, the addition of ascorbic acid deters the formation of the technetium-99m/protein complex. It is believed that the ascorbic acid forms a "strong" complex with the $^{99m}Tc$ having a strength such that the proteins and cells cannot remove the technetium-99m from the ascorbic acid/technetium-99m complex to form a technetium-99m protein complex.

It is believed that the reason ascorbic acid and dihydroxybenzoic acid are equivalent in the formation of phosphorus complexes for bone scintigraphy is that the phosphorus/technetium-99m complex is stronger than either the dihydroxybenzoic acid or ascorbic acid complex.

In summary, while there has been prior use of dihydroxybenzoic acid as a stabilizer of stannous ions in the formation of technetium-99/phosphorus complexes, such use has not made the use of the dihydroxybenzoic acid as a mediator or catalyst in the formation of technetium-99m/protein complexes obvious. While those skilled in the art have attempted to label proteins which technetium-99m for years, the inability to form such complexes with the use of the "equivalent" ascorbic acid have led those skilled in the art away from the use of the benzoic acid derivatives.

The preferred concentration ranges for the components used in the present invention are as follows: stannous ions—about 40 µmol/l; benzoic acid derivative—about 5 mmol/l; technetium-99m—about 15 mCi/ml; and protein—about 3 mg/ml. Sodium chloride and hydrochloric acid and sodium hydroxide are used to adjust the osmotic pressure and pH as tolerated by the proteins or cells.

The invention is further illustrated with reference to the following specific Examples of embodiments of the invention, which are illustrative and not limiting.

It should be noted that Examples 1—3 do not exemplify the invention, but illustrate chemical composition suitable for Tc-labelling of cells.

Example 1

An aqueous solution containing 5 mmol/l of 2,5-hydroxybenzoic acid and 40 µmol/l of stannous citrate is prepared. The pH is adjusted to neutral and the solution is sterilized by filtration and dispensed in aliquot portions of 0.5 ml, which are freeze-dried in sterile vials. The procedure is carried out with sterile pyrogen-free reagents by means of aseptic techniques and under a nitrogen atmosphere. The dry solids of one vial are dissolved in 0.5 ml of an aqueous solution of pertechnetate and 0.9% NaCl, obtained as eluate from a $^{99m}$Tc/$^{99}$Mo-generator. Granulocytes, isolated from 50 ml human blood, are suspended in the resulting solution, and the suspension is maintained for 30 minutes by gently moving the liquid. The cells are then isolated by addition of 2 ml plasma from the same person and centrifuged. After removal of the supernatant the cells are resuspended in 2 ml plasma before the suspension is injected into the patient or the organism which is to be examined. Thereby a radiochemical purity of more than 90% is achieved for the technetium-99m present, which is bonded to the granulocytes. The total amount of technetium-99m bonded to all the granulocytes is suitable for scintigraphic depiction. The amount of 99m technetium bonded to the cells will vary with the amount of technetium-99m in the initial solution (the generator eluate). Human studies with autologous technetium-99m labelled granulocytes prepared according to this method show a biodistribution corresponding to the biodistribution of 111-In-labelled granulocytes and with a minor urine-secreted amount of the injected technetium-99m during the first hours. Corresponding or even better results are obtained when stannous tartrate is used instead of stannous citrate. Similar results were obtained when 2,5-dihydroxy-benzoic acid was replaced by 2,3-dihydroxy-benzoic acid, 3,4-dihydroxy-benzoic acid, 4-aminobenzoic acid or 2-, 3- or 4-hydroxy-benzoic acid.

Example 2

Blood platelets (thrombocytes) isolated from human blood are labelled with technetium-99m in the same way as in Example 1. However, the granulocytes are replaced by blood platelets isolated from 40 ml human blood. After washing of the cells as stated in Example 1 a high radiochemical purity more than 90% of the present technetium-99m bonded to the blood platelets was achieved. The total amount of technetium-99m bonded to the blood platelets is suitable for scintigraphic depiction. The amount of technetium-99m bonded to the cells will vary with the amount of technetium-99m in the initial solution (the generator eluate). Human studies with autologous technetium-99m labelled blood platelets prepared according to this procedure have shown a biodistribution corresponding to that of 111-In-labelled blood platelets and with a corresponding minor urine-secreted amount of technetium-99m during the first hours after the injection as in Example 1. Similar to Example 1, the tartrate can be used with good result, and similarly the benzoic acid derivatives mentioned in Example 1.

Example 3

Leukocytes in the form of a fraction containing all the types of leukocytes, isolated from human blood, are labelled with technetium-99m as specified in Example 1. However, the granulocytes are replaced by the leukocytes isolated from 30 ml human blood. Details regarding the washing or separation of the labelled cells and the obtained radiochemical purity do not deviate substantially from those of Examples 1 and 2. A labelling of the cells is obtained with an amount of technetium-99m well suitable for scintigraphic depiction. As in Examples 1 and 2 the tartrate is used with good result, and the same applied to the use of the benzoic acid derivatives mentioned in Example 1.

Example 4

An aqueous solution of 9 mmol/l of 2,5-dihydroxybenzoic acid and 55 µmol/l stannous chloride is prepared. The pH is adjusted to 3.5 and rabbit immunoglobulin G dissolved in 0.150 mole/l aqueous solution is added to 2 mg per ml. Sterilization by filtration is carried out and aliquot portions of 0.5 ml are dispensed and freeze-dried in sterile vials by means of aseptic technique and under nitrogen atmosphere. The dry solid in a vial is dissolved in 0.5 ml pertechnetate solution in 0.9% NaCl solution, as eluate from a $^{99}$Tc/$^{99}$Mo-generator. After a period of 40 minutes, technetium-99m bonded to the immunoglobulin having a high radiochemical purity, typically 90—98%, is achieved. Similar results were obtained when 2,5-hydroxybenzoic acid was replaced by the benzoic acid derivatives mentioned in Example 1.

Example 5

An aqueous solution of 8 µmol/l of 2,5-dihydroxybenzoic acid and 55 µmol/l of stannous citrate is prepared. The pH is adjusted to 5.0 and rabbit immunoglobulin G dissolved in 0.150 mole/l aqueous NaCl solution is added to 2 mg per ml. Sterilization of filtration is carried out and 0.5 ml aliquot portions are dispensed and freeze-dried in sterile vials with use of aseptic techniques and under nitrogen atmosphere. The dry solid in a vial is dissolved in 0.5 ml pertechnetate solution 0.9% NaCl aqueous solution, as eluate from a 99m technetium-$^{99m}$/$^{99}$Mo-generator. After 40 minutes technetium-99 bonded to the immunoglobulin is achieved having a high radiochemical purity. Judged by means of radioimmunoelectrophoresis the antigenicity and the immunoaffinity of the labelled immunoglobulins are maintained. Also here stannous tartrate may be used instead of stannous citrate, with results which cannot be distinguished from the results by use of stannous citrate, and the same applies to the use of the benzoic acid derivatives mentioned in Example 1.

Example 6

A solution is prepared as described in Example 4. However, rabbit immunoglobulin is not used. Instead murine monoclonal immunoglobulin having affinity for carcinoembryonic antigen prepared by hybridom technique is used. The remainder of the Example is identical with Example 4 with corresponding technetium-99m labelling result.

Example 7

In the foregoing Examples of labelling of cells (Example 1—3) the labelling process has been carried out with the following sequence:

a) preparation of reagent component (stannous salt+benzoic acid derivatives mentioned in Example 1)

b) addition of pertechnetate solution

c) admixing of cells, and

d) washing or centrifugal separation of the labelled cells.

For the labelling of granulocytes and leukocytes, it has been shown that the following sequence gives similar or improved results.

a) preparation of reagent component (stannous salt+benzoic acid derivative mentioned in Example 1)

b) admixing of cells for diffusion into the cells of the reagents from a)

c) washing or centrifugal separation for removal of reagents which have not been taken up by the cells

d) addition of pertechnetate for diffusion into the cells so that the labelling reaction can occur theretil, and

e) washing or centrifugal separation of the labelled cells.

Example 8

An aqueous solution consisting of 8 μmol/l of 2,5-hydroxy-benzoic acid and 55 μmol/l of stannous chloride per liter is prepared. The pH is adjusted to 3.5. Monoclonal immunoglobulin with specific affinity for human leukocytes, dissolved in 0.150 moles per liter NaCl, is added to 2 milligrams per milliliter. The solution is sterilized by filtration and aliquot portions of 0.5 ml are dispensed and freeze-dried in sterile vials by means of aseptic techniques and under nitrogen atmosphere. The dry solid in a vial is dissolved in 0.5 ml of pertechnetate solution. After a period of thirty minutes technetium-99m bonded to the immunoglobulin having a high radiochemical purity, typically 90—98%, is achieved. The leukocytes of the patient are labelled indirectly with technetium-99m by intravenous injection of the immunoglobulin solution or by mixing the immunoglobulin solution with blood of the patient which is then intravenously injected. The radioactivity corresponding to the distribution of the labelled cells is such that detection and depiction of the inflammatory and infectious diseases or rejection reactions where these leukocytes play a role can be obtained by scintigraphy or other external radiometric measurements. The stannous chloride may be replaced by stannous citrate or stannous tartrate. Similar results were obtained when 2,5-dihydroxy-benzoic acid was replaced by the benzoic acid derivatives mentioned in Example 1.

Example 9

Granulocytes are labelled with the technetium-99m as specified in Example 8, however, the monoclonal immunoglobulins with specific affinity for human leukocytes are replaced with monoclonal immunoglobulins with specific affinity for human granulocytes.

Example 10

Lymphocytes are labelled with technetium-99m as specified in Example 8, however, the monoclonal immunoglobulins with specific affinity for leukocytes are replaced by immunoglobulins with specific affinity for human lymphocytes.

Example 11

Platelets are labelled with technetium-99m as specified in Example 8, however, the monoclonal immunoglobulins with specific affinity for human leukocytes are replaced with immunoglobulins with specific affinity for platelets. The labelled platelets permit detection and depiction of thrombosis and embolic disease.

The present invention may be embodied in other specific forms.

**Claims**

1. A chemical composition for use in $^{99m}$Tc labelling of protein or cells comprising proteins or cells to be labelled, the amount of protein as such or in the form of cell protein being 0.25 to 10 mg/ml, from 0.5 to 50 mmol/l of a benzoic acid derivative that is an aminobenzoic, monohydroxybenzoic or dihydroxybenzoic acid or a water-soluble salt thereof, and a stannous salt in an amount to provide 4 to 150 μmol/l of stannous ions.

2. A composition according to Claim 1 further comprising 0.5 to 30 mCi/ml of technetium-99m ions formed from an added pertechnetate solution.

3. A composition according to Claim 1 or 2, in which the benzoic acid derivative is 2,3-, 2,5- or 3,4-dihydroxybenzoic acid or a salt thereof.

4. A composition according to Claim 1 or 2, in which the benzoic acid derivative is 4-aminobenzoic acid or a salt thereof.

5. A composition according to Claim 1 or 2, in which the benzoic acid derivative is 2, 3- or 4-hydroxybenzoic acid or a salt thereof.

6. A composition according to any one of Claims 1 to 5, in which the protein is an immunoglobulin.

7. A composition according to any one of Claims 1 to 5, in which the cells are leukocytes, thrombocytes and/or erythrocytes.

8. A composition according to Claim 6, in which the protein is an immunoglobulin with specific affinity for leukocytes.

9. A composition according to Claim 8, in which the immunoglobulin is an immunoglobulin having specific affinity for granulocytes and/or an immunoglobulin having specific affinity for lymphocytes.

10. A composition according to any one of Claims 1 to 9, in which the stannous salt is a stannous halide or a stannous salt of a carboxylic acid.

11. A composition according to any one of Claims 1 to 9, in which the stannous salt is stannous chloride, stannous tartrate or stannous citrate.

12. A composition according to any one of Claims 1 to 11 further comprising a tartrate salt or citrate salt other than stannous tartrate and stannous citrate.

13. A composition according to Claim 12, in which the said salt is sodium tartrate or sodium citrate.

14. A composition according to any one of Claims 1 to 13 having a pH and ionic strength tolerated by the protein or cells to be labelled.

15. A composition in solid form according to any one of Claims 1 to 6, 8 and 9, comprising proteins to be labelled but not cells.

16. A composition according to Claim 15 in a freeze-dried form.

## Patentansprüche

1. Chemische Komposition zur Verwendung beim $^{99m}$Tc-Markieren von Protein oder Protein enthaltenden Zellen oder zu markierenden Zellen, wobei der Proteingehalt als solcher oder in der Form von Zellprotein 0,25 bis 10 mg/ml ausmacht, bestehend aus 0,5 bei 50 nmol/l eines Benzoesäurederivates in Form von Aminobenzoesäure, Monohydroxybenzoesäure oder Dihydroxybenzoesäure oder eines wasserlöslichen Salzes desselben und einem Zinnsalz in eine 4 bis 150 nmol/l Zinnionen liefernden Menge.

2. Komposition nach Anspruch 1, enthaltend weiterhin 0,5 bis 30 mCi/ml Technetium-99m—Ionen, die aus einer zugefügten Pertechnetatlösung gebildet sind.

3. Komposition nach Anspruch 1 oder 2, bei der das Benzoesäurederivat 2,3-, 2,5- oder 3,4-Dihydroxybenzoesäure oder ein Salz derselben ist.

4. Komposition nach Anpsruch 1 oder 2, bei der das Benzoesäurederivat 4-Aminobenzoesäure oder ein Salz derselben ist.

5. Komposition nach Anspruch 1 oder 2, bei der das Benzoesäurederivat 2-, 3- oder 4-Hydroxybenzoesäure oder ein Salz derselben ist.

6. Komposition nach einem der Ansprüche 1 bis 5, bei der das Protein Immunoglobulin ist.

7. Komposition nach einem der Ansprüche 1 bis 5, bei der die Zellen Leukozyten, Thrombozyten und/oder Erythrozyten sind.

8. Komposition nach Anspruch 6, bei der das Protein ein Immunoglobulin mit spezifischer Affinität für Leukozyten ist.

9. Komposition nach Anspruch 8, bei der das Immunoglobulin ein Immunoglobulin mit spezifischer Affinität für Granulozyten und/oder ein Immunoglobulin mit spezifischer Affinität für Lymphozyten ist.

10. Komposition nach einem der Ansprüche 1 bis 9, bei der das Zinnsalz ein Zinnhalogenid oder ein Zinnsalz einer Carbonsäure ist.

11. Komposition nach einem der Ansprüche 1 bis 9, bei der das Zinnsalz Zinnchlorid, Zinntartrat oder Zinnzitrat ist.

12. Komposition nach einem der Ansprüche 1 bis 11, enthaltend weiterhin ein von Zinntartrat und Zinnzitrat abweichendes Tartrat- oder Zitratsalz.

13. Komposition nach Anspruch 12, bei der das Salz Natriumtartrat oder Natriumzitrat ist.

14. Komposition nach einem der Ansprüche 1 bis 13, welche einen pH-Wert und eine Ionenstärke aufweist, die vom Protein oder den zu markierenden Zellen toleriert wird.

15. Komposition in fester Form nach einem der Ansprüche 1 bis 6, 8 und 9, enthaltend zu markierende Proteine, aber nicht Zellen.

16. Komposition nach Anspruch 15 in einer gefriergetrockneten Form.

## Revendications

1. Composition chimique destinée à être utilisée pour le marquage avec $^{99m}$Tc d'une protéine ou de cellules, comprenant des protéines ou des cellules destinées à être marquées, la quantité de protéine telle quelle ou sous la forme de protéine cellulaire étant de 0,25 à 10 mg/ml, de 0,5 à 50 mmoles/l d'un dérivé d'acide benzoïque, qui est un acide aminobenzoïque, monohydroxybenzoïque ou dihydroxybenzoïque, ou un sel soluble dans l'eau de celui-ci, et un sel stanneux selon une quantité suffisante pour procurer une quantité de 4 à 150 µmoles/l d'ions stanneux.

2. Composition selon la revendication 1, comprenant en outre de 0,5 à 30 mCi/ml d'ions technétium 99m, formés à partir d'une solution de pertechnétate additionnée.

3. Composition selon la revendication 1 ou 2, dans laquelle le dérivé d'acide benzoïque, est l'acide 2,3-, 2,5- ou 3,4-dihydroxybenzoïque, ou un sel de celui-ci.

4. Composition selon la revendication 1 ou 2, dans laquelle le dérivé d'acide benzoïque, est l'acide 4-aminobenzoïque, ou un sel de celui-ci.

5. Composition selon la revendication 1 ou 2, dans laquelle le dérivé d'acide benzoïque, est l'acide 2,3- ou 4-hydroxybenzoïque, ou un sel de celui-ci.

6. Composition selon l'une quelconque des revendications 1 à 5, dans laquelle la protéine est une immunoglobuline.

7. Composition selon l'une quelconque des revendications 1 à 5, dans laquelle les cellules comprennent des leucocytes, des thrombocytes et/ou des érythrocytes.

8. Composition selon la revendication 6, dans laquelle la protéine est une immunoglobuline ayant une affinité spécifique pour les leucocytes.

9. Composition selon la revendication 8, dans laquelle l'immunoglobuline est une immunoglobuline ayant une affinité spécifique pour les granulocytes et/ou une immunoglobuline ayant une affinité spécifique pour les lymphocytes.

10. Composition selon l'une quelconque des revendications 1 à 9, dans laquelle le sel stanneux est un halogénure stanneux ou un sel stanneux dérivé d'un acide carboxylique.

11. Composition selon l'une quelconque des revendications 1 à 9, dans laquelle le sel stanneux est le chlorure stanneux, le tartrate stanneux ou le citrate stanneux.

12. Composition selon l'une quelconque des revendications 1 à 11, comprenant en outre un sel de type tartrate ou un sel de type citrate, autre que du tartrate stanneux et du citrate stanneux.

13. Composition selon la revendication 12, dans laquelle ledit sel est du tartrate de sodium ou du citrate de sodium.

14. Composition selon l'une quelconque des revendications 1 à 13, ayant un pH et une force ionique, tolérés par la protéine ou les cellules destinées à être marquées.

15. Composition sous forme solide, selon l'une quelconque des revendications 1 à 6, 8 et 9, comprenant des protéines destinées à être marquées, mais pas de cellules.

16. Composition selon la revendication 15, sous forme lyophilisée.